# EUROPEAN PATENT APPLICATION

(11) **EP 4 378 961 A1**
(43) Date of publication of application: **05.06.2024**
(21) Application number: 22857592.4
(22) Date of filing: 03.08.2022
(51) Int. Cl.: C07K 16/46, C12N 15/13, A61K 39/395, A61P 31/14

(54) **FULLY HUMAN SINGLE DOMAIN ANTIBODY-BASED ANTI-SARS-COV-2 BISPECIFIC NEUTRALIZING ANTIBODY AND USE THEREOF**

(30) Priority: 19.08.2021 CN 202110952739
(71) Applicant: Biomissile (Anji) Pharmaceuticals Co., Ltd., Zhejiang 313000 (CN)
(72) Inventor: WU, Yanling, Shanghai 200433 (CN); LI, Cheng, Shanghai 200433 (CN)
(74) Representative: Sun, Yiming
(86) International application number: PCT/CN2022/109969
(87) International publication number: WO 2023/020280

(57) **Abstract**

Disclosed are a fully human bispecific antibody against SARS-CoV-2 and a use thereof. Specifically, the present invention also relates to a vector and host cell expressing same, and a preparation and purification method therefor. The bispecific antibody has a broad-spectrum binding ability and neutralizing ability, and the bispecific antibody can be used in the preparation of drugs for treating or preventing diseases caused by SARS-CoV-2 infection and in detection or diagnosis of COVID-19.

## Description

### TECHNICAL FIELD

The present invention belongs to the field of biotechnology, and in particular relates to a bispecific antibody that binds to SARS-CoV-2, a preparation method therefor and use thereof.

### BACKGROUND

COVID-19 is pneumonia caused by SARS-CoV-2 infection. Most of clinical manifestations are mild, and severe cases can lead to death. Currently, SARS-CoV-2 strains from a source of COVID-19 pandemic have been gradually replaced by some SARS-CoV-2 variants that are highly infectious and spread at a high speed, including B.1.1.7 (201/501YV1) in the UK, B.1.351 (20H/501.V2) in South Africa, P.1 (20J/501Y.V3) in Brazil, and B.1.617.1 (20A/S:154K) and B.1.617.2 (21A/S:478K) in India. Most importantly, these variants can not only escape the vast majority of neutralizing antibodies, but also weaken the protective ability of existing vaccines, thereby spreading widely in various countries and regions around the world. Therefore, there is an urgent need to develop a broad-spectrum therapeutic biological agent against SARS-CoV-2 and its variants.

Bispecific antibodies have obvious advantages over single antibodies in broad spectrum and neutralizing activity due to their simultaneous targeting of two different epitopes, thus having great potential as therapeutic drugs.

SARS-CoV-2 invasion is mediated through the binding of a receptor binding domain (RBD) on spike glycoprotein on the viral surface to human angiotensin-converting enzyme 2 (ACE2) on the cell surface. Therefore, the RBD is a key target for neutralizing antibodies. Currently, most of fully human monoclonal antibody drugs against SARS-CoV-2 that have been approved for marketing and are being studied clinically target the RBD. However, a molecular weight of a monoclonal antibody is too large (about 150 kDa), resulting in high production cost and high price of the monoclonal antibody drugs, preventing its large-scale clinical application in the prevention and treatment of infectious diseases. For a long time, people have been exploring to replace monoclonal antibodies in the form of IgG with "antibody fragments" with smaller molecular weight and soluble expression in prokaryotic cells, as a new generation of antibody drugs with low production cost and high tissue permeability. In recent years, a class of antibodies containing only a variable region of a heavy chain has been found in alpaca serum, is the smallest antibody fragment having antigen-binding properties in nature, and is called nanobodies (VHH). These nanobodies have high antigen affinity and specificity, are similar to full-length IgG monoclonal antibodies, and are easier to engineer and can be expressed in prokaryotic systems, and are therefore inexpensive to prepare. Because of a small molecular weight, excellent solubility, stability and other characteristics, the nanobodies can be administered by inhalation, so that the nanobodies can reach the lungs directly, take effect quickly, and are high in usability, and are very suitable for being used as a lung delivery preparation to treat respiratory system diseases.

Thus, those skilled in the art hope to develop a highly active bispecific antibody that neutralizes a variety of SARS-CoV-2 mutants and escape mutants in a broad spectrum, so as to effectively prevent or treat SARS-CoV-2 infection.

### SUMMARY

In order to solve the above technical problem, the present invention provides a bispecific neutralizing antibody against SARS-CoV-2. The bispecific antibody includes two single domain antibodies sdAb-1 and sdAb-2 that can bind to spike glycoprotein of SARS-CoV-2, wherein the sdAb-1 has an amino acid sequence shown in SEQ ID NO: 1, and the sdAb-2 has an amino acid sequence shown in SEQ ID NO: 2.

Preferably, a C-terminus of the sdAb-1 is linked to a N-terminus of the sdAb-2 by a peptide linker.

Preferably, the peptide linker has a sequence of (Gly4Ser)4, as shown in SEQ ID NO: 3.

Preferably, the bispecific antibody includes the amino acid sequence shown in SEQ ID NO: 1 and/or SEQ ID NO: 2 or a sequence having at least 90%, 91%, 92%, 93%, 94%, 95%, 96%, 97%, 98% or 99% identity to the amino acid sequence.

Preferably, each single domain antibody is a variable region of a heavy chain (VH).

Preferably, the variable region of the heavy chain (VH) is fully human.

The present invention also provides a fusion protein, including the bispecific antibody described above fused to a heterologous protein.

Preferably, the heterologous protein is Fc of human immunoglobulin, more preferably Fc of human IgG1.

The present invention also provides a conjugate, including the bispecific antibody described above, or any one of the fusion proteins described above, which is conjugated to an effector molecule.

Preferably, the effector molecule is a detectable label.

Preferably, the detectable label is a fluorescent label, a radiolabel, avidin, biotin, or an enzyme.

The present invention provides a nucleic acid encoding the bispecific antibody described above. The present invention provides a vector including the nucleic acid, wherein optionally, the vector is operably linked to a regulatory sequence. The present invention provides a host cell including the vector, and a method for producing and optionally recovering the bispecific antibody or antigen binding fragments. The host cell of the present invention can be any prokaryotic or eukaryotic cell, including but not limited to bacterial cells (e.g., Escherichia coli), insect cells (e.g., using a baculovirus expression system), and yeast or mammalian cells (e.g., CHO or BHK cell lines). Other suitable host cells are known to those skilled in the art. Preferred host cells are Escherichia coli cells, which have a short culture cycle and low production cost.

The bispecific antibody provided by the present invention can specifically bind to corresponding antigens which refer to all substances that can induce an immune response in the body. The antigens include, but are not limited to, small molecule compounds. The antigens include, but are not limited to, proteins listed below, and subunits, domains, motifs and epitopes belonging to the following proteins: CD2, CD3, CD3E, CD4, CD11, CD11a, CD14, CD16, CD18, CD19, CD20, CD22, CD23, CD25, CD28, CD29, CD30, CD32, CD33 (p67 protein), CD38, CD40, CD40L, CD52, CD54, CD56, CD80, CD147, GD3, IL-1, IL-1R, IL-2, IL-2R, IL-4, IL-5, IL-6, IL-6R, IL-8, IL-12, IL-15, IL-18, IL-23, α-interferon, β-interferon, γ-interferon; TNF-α, TNFβ2, TNFc, TNfαβ, TNF-RI, TNF-RII, FasL, CD27L, CD30L, 4-1BBL, TRAIL, RANKL, TWEAK, APRIL, BAFF, LIGHT, VEGI, OX40L, TRAIL receptor-1, an A1 adenosine receptor, a lymphotoxin beta receptor, TACI, BAFF-R, EPO; IFA-3, ICAM-1, ICAM-3, EpCAM, β1-integrin, β2-integrin, α4/β7-integrin, α2-integrin, α3-integrin, α4-integrin, α5-integrin, α6-integrin, αv-integrin, αVβ3-integrin, FGFR-3, a keratinocyte growth factor, VLA-1, VLA-4, L-selectin, an anti-idiotypic antibody (anti-id), E-selectin, HLA, HLA-DR, CTLA-4, a T cell receptor, B7-1, B7-2, VNR integrin, TGF-β1, TGF-β2, eotaxin 1, BLyS (a B-lymphocyte stimulator), a complement C5, IgE, a factor VII, CD64, CBL, NCA 90, EGFR (ErbB-1), Her2/neu (ErbB-2), Her3 (ErbB-3), Her4 (ErbB-4), a tissue factor, VEGF, VEGFR, an endothelin receptor, VLA-4, a hapten NP-capping or NIP-capping protein, a T cell receptor α/β, E-selectin, digoxin, placental alkaline phosphatase (PLAP) and testicular PLAP-like alkaline phosphatase, a transferrin receptor, a carcinoembryonic antigen (CEA), CEACAM5, HMFG PEM, mucin MUC1, MUC18, heparanase I, human cardiac myosin, tumor-associated glycoprotein-72 (TAG-72), a tumor-associated antigen CA125, a prostate specific membrane antigen (PSMA), a high molecular weight melanoma-associated antigen (HMW-MAA), a cancer associated antigen, Gcoprotein IIb/IIIa (GPIIb/IIIa), a tumor-associated antigen expressing Lewis Y related carbohydrate, human cytomegalovirus (HCMV) gH envelope glycoprotein, HIV gp120, HIV gp140, HCMV, respiratory syncytial virus RSVF, RSVF Fgp, VNR integrin, IL-8, a cytokeratin tumor associated antigen, Hep B gp120, CMV, gpllbllla, an HIV IIIB gp120 V3 loop, respiratory syncytial virus (RSV) Fgp, herpes simplex virus (HSV) gD glycoprotein, HSV gB glycoprotein, HCMV gB envelope glycoprotein, and Clostridium perfrmgens toxin, and programmed death-1 (PD-1).

Those skilled in the art should understand that the above-listed targets refer not only to specific proteins and biomolecules, but also to biochemical pathway or various pathways including the targets. For example, when CTLA-4 mentioned is used as a target antigen, it is meant that a ligand and a receptor constitute a T cell co-stimulatory pathway, including CTLA-4, B7-1, B7-2, and CD28, and any other undiscovered ligands or receptors are also target objects. Therefore, the target as used herein refers not only to a specific biological molecule, but also to a group of proteins that interact with the target and members of a biochemical pathway to which the target belongs. Those skilled in the art should further understand that any of the above-described target antigens, ligands or receptors linked to the target antigens, or other members of their corresponding biochemical pathways can be operatively linked to the single domain antibodies or antigen binding fragments of the present invention so as to generate fusion bodies. Thus, virtually, any polypeptide, whether a ligand, a receptor or some other proteins or a protein domain, including but not limited to the above-mentioned targets and proteins that constitute their corresponding biochemical pathways, can be operably linked to the single domain antibodies or antigen binding fragments of the present invention to form fusion bodies.

More preferably, a source of the antigens includes, but is not limited to, cancer, infectious diseases (e.g., viral, bacterial, fungal, and parasitic infections, etc.), autoimmune diseases, and inflammatory disorders.

The antigens may be from viruses. The viruses described in the present invention are, for example, viruses from one of the following families: retroviridae (e.g., human immunodeficiency virus (HIV), and human T-cell leukemia virus (HTLV)); picornaviridae (e.g., poliovirus, hepatitis A virus, hepatitis C virus, enterovirus, human coxsackievirus, rhinovirus, echovirus, and foot-and-mouth disease virus); cadmyxoviridae (such as strains that cause gastroenteritis); togaviridae (e.g., Equine encephalitis virus, and rubella virus); flaviviridae (e.g., Dengue virus, yellow fever virus, West Nile virus, St. Louis encephalitis virus, Japanese encephalitis virus, and other encephalitis viruses); coronaviridae (e.g., coronavirus, and severe acute respiratory syndrome (SARS) virus); rhabdoviridae (e.g., vesicular stomatitis virus, and rabies virus); paramyxoviridae (e.g., parainfluenza virus, mumps virus, measles virus, respiratory syncytial virus (RSV)); orthomyxoviridae (e.g., influenza virus); bunyaviridae (e.g., Hantaan virus, Sin Nombre virus, Rift Valley Fever virus, bunya virus, phleboviruses and Nairo virus); arenaviridae (e.g., hemorrhagic fever virus, Machupo virus, and Junin virus); reoviridae (e.g., reovirus, orbiviurse and rotavirus); birnaviridae; hepadnaviridae (e.g., hepatitis B virus); parvoviridae (e.g., parvovirus); papovaviridae (e.g., papillomavirus, polyoma virus, and BK virus); adenoviridae (e.g., most adenoviruses such as adeno-associated virus); herpesviridae (e.g., herpes simplex viruses (HSV-1 and HSV-2), cytomegalovirus (CMV), Epstein-Barr virus (EBV), varicella zoster virus (VZV), and other herpes viruses, including HSV-6); poxviridae (e.g., variola virus, vaccinia virus, and pox virus); and Iridoviridae (such as African swine fever virus); viridae (e.g., Ebola virus, and Marburg virus); caliciviridae (e.g., Norwalk virus) and unclassified viruses (e.g., a pathogen of spongiform encephalopathy, a pathogen of delta hepatitis (considered to be a defective satellite of hepatitis B virus), and astrovirus).

The antigens may be from bacteria. The bacteria described in the present invention are, for example, Helicobacter pylori, Borelia burgdorferi, Legionella pneumophila, Mycobacteria (such as M. tuberculosis, M. avium, M. intracellulare, M. kansaii, and M. gordonae), Staphylococcus aureus, Neisseria gonorrhoeae, neisseria meningitidis, listeria monocytogenes, streptococcus pyogenes (Group A streptococcus), streptococcus agalactiae (Group B streptococcus) , Streptococcus (a population of grass green flies) , Streptococcus faecalis, streptococcus bovis, streptococcus (anaerobe) , Streptococcus pneumoniae, Pathogenic Campylobacter, Enterococcus, Haemophilus influenzae, Bacillus anthracis, Corynebacterium diphtheriae, Corynebacterium, swine fever virus, Clostridium perfringens, Clostridium tetani, Enterobacter aerogenes, Klebsiella pneumoniae, Pasteurella multocida, Bacteroides, Clostridium, malt streptomyces, Treponema pallidum, Treponema, Leptospira, or Actinomyces israelli.

The antigens may be from fungi. The fungi described in the present invention are, for example, Cryptococcus neoformans, Histoplasma capsulatum, Coccidioides immitis, Blastomyces dermatitidis, Chlamydia trachomatis or Candida albicans.

The antigens may be from parasites. The parasites described in the present invention are, for example, Plasmodium falciparum or Toxoplasma gondii.

The antigens may be cancer antigens, such as solid tumor or blood-borne cancer antigens. The solid tumor described in the present invention is sarcoma or carcinoma, e.g., fibrosarcoma, myxosarcoma, liposarcoma, chondrosarcoma, osteogenic sarcoma, or another sarcoma, synovioma, mesothelioma, Ewing's tumor, leiomyosarcoma, rhabdomyosarcoma, colon cancer, lymphoid malignancy, pancreatic cancer, breast cancer, lung cancer, ovarian cancer, prostate cancer, hepatocellular carcinoma, squamous cell carcinoma, basal cell carcinoma, adenocarcinoma, sweat gland carcinoma, sebaceous gland carcinoma, papillary carcinoma, papillary adenocarcinoma, medullary carcinoma, bronchial carcinoma, renal cell carcinoma, hepatocellular carcinoma, cholangiocarcinoma, choriocarcinoma, Wilms' tumor, cervical cancer, testicular tumor, bladder cancer or a tumor of a central nervous system (such as glioma, astrocytoma, medulloblastoma, craniopharyngioma, ependymoma, pineal gland, hemangioblastoma, acoustic neuroma, oligodendroglioma, hemangioma, melanoma, neuroblastoma or retinoblastoma). The blood-borne cancer described in the present invention is leukemia, such as acute leukemia (such as acute lymphocytic leukemia, acute myelocytic leukemia, acute myeloid leukemia and myeloblastic leukemia, promyelocytic leukemia, myelomonocytic leukemia, monocytic leukemia and erythroleukemia); chronic leukemia (such as chronic myelocytic (granulocytic) leukemia, chronic myelocytic leukemia and chronic lymphocytic leukemia), polycythemia vera, lymphoma, Hodgkin's disease, non-Hodgkin's lymphoma (indolent and advanced), multiple myeloma, Waldenstrom's macroglobulinemia, heavy chain disease, myelodysplastic syndrome, hairy cell leukemia, or myelodysplasia. Tumor antigens are well known in the art, including, for example, a carcinoembryonic antigen (CEA), β-human chorionic gonadotropin (β-HCG), alpha-fetoprotein (AFP), lectin-reactive AFP, (AFP-L3), thyroglobulin, RAGE-1, MN-CA IX, human telomerase reverse transcriptase (hTERT), RU1, RU2 (AS), intestinal carboxyesterase, mut hsp70-2, M-CSF, a prostaglandin enzyme, a prostate-specific antigen (PSA), PAP, NY-ESO-1, LAGE-1a, p53, prostein, PSMA, Her2/neu, survivin and telomerase, prostate cancer tumor antigen-1 (PCTA-1), a melanoma-associated antigen (MAGE), ELF2M, neutrophil elastase, ephrinB2, and CD22. The tumor antigens can also be any cancer associated protein, such as IGF-I, IGF-II, IGR-IR or mesothelin.

The present invention provides a pharmaceutical composition, prepared by mixing an effective prophylactic or therapeutic dose of the bispecific antibody, the fusion protein, the conjugate, the nucleic acid molecule of the bispecific antibody, or the vector including the nucleic acid molecule of the bispecific antibody according to the present invention with a physiologically or pharmaceutically acceptable carrier, excipient or stabilizer, and the composition includes, but is not limited to, a lyophilized dosage form, an aqueous solution dosage form, a liposome or capsule dosage form, and the like. The concentration of the bispecific antibody, the fusion protein, the conjugate, the nucleic acid molecule of the bispecific antibody, and the vector including the nucleic acid molecule of the bispecific antibody according to the present invention may vary from about 0.1% to 100% by weight.

### BRIEF DESCRIPTION OF THE DRAWINGS

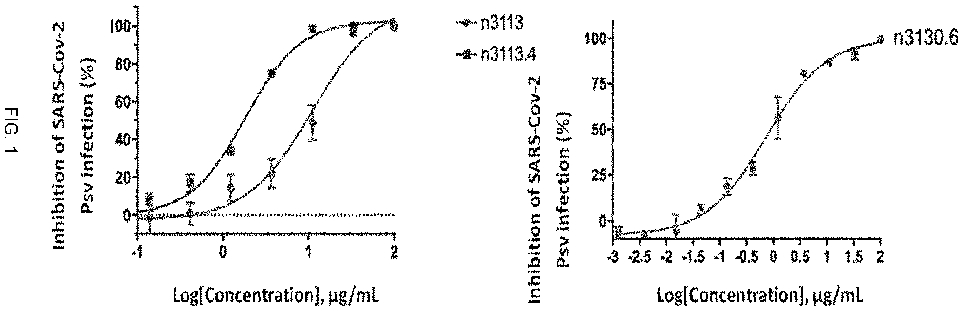

FIG. 1: Neutralizing activity of single domain antibodies n3113.4 and n3130.6 after affinity maturation against SARS-CoV-2 pseudovirus.
FIG. 2: Structural schematic diagram of a bispecific antibody against SARS-CoV-2.
FIG. 3: Detection of the purity of a bispecific antibody by sodium dodecyl sulfate-polyacrylamide gel electrophoresis (SDS-PAGE).
FIG. 4: Detection of the binding ability of a bispecific antibody to an RBD protein of a SARS-CoV-2 variant by ELISA.
FIG. 5: Detection of the binding affinity of a bispecific antibody to an RBD protein of a SARS-CoV-2 variant B1.617 by using ForteBio.
FIG. 6: Detection of the neutralizing activity of single domain antibodies n3113.4 and n3130.6 and a double antibody against an original strain of SARS-CoV-2 and a variant strain B1.617 by using a pseudovirus system, wherein a monoclonal antibody S309 is used as a positive control.

### DETAILED DESCRIPTION

In order to understand the present invention more thoroughly, some definitions are listed below. The above definitions are intended to include grammatical equivalents.

A "bispecific antibody" used herein means an artificial antibody that contains two specific antigen-binding sites.

An "antibody molecule" herein means a protein consisting of one or more domains substantially encoded by all or part of recognized immunoglobulin genes. The recognized immunoglobulin genes, e.g., in humans, include kappa (κ), lambda (λ) and heavy chain loci, which contain numerous variable region genes, as well as constant region genes mu (µ), delta (δ), gamma (γ), epsilon (ε), and alpha (a) which encode IgM, IgD, IgG, IgE and IgA isotypes, respectively. Antibodies herein are meant to include full-length antibodies, individual chains thereof, as well as all portions, domains or fragments thereof, as well as natural antibodies from any organism, engineered antibodies, or antibodies recombinantly produced for experimental and therapeutic purposes, or other purposes as further specified below. The term "antibody" includes antibody fragments known in the art, such as Fab, Fab', F(ab')2, Fv, scFv, or antigen binding domains of antibodies (e.g., VHH domains or VH/VL domains), or antibody fragments produced by the modification of intact antibodies or those synthesized de novo by using a recombinant DNA technology. The term "antibody" includes a monoclonal antibody and a polyclonal antibody. The antibody may be an antagonist, an agonist, a neutralizing antibody, or an inhibitory antibody, or a stimulatory antibody. The antibody of the present invention may be a non-human antibody, a chimeric antibody, a humanized antibody or a fully human antibody.

A non-glycosylated antibody is specifically included within the definition of the "antibody". Preferably, the "non-glycosylated antibody" used herein means an antibody lacking sugar attachment at a position 297 of a Fc region, wherein numbering is performed according to an EU system of Kabat. The non-glycosylated antibody may be a deglycosylated antibody, which is an antibody from which Fc sugar has been removed, for example chemically or enzymatically. Optionally, the non-glycosylated antibody may be a non-glycosylated or aglycosylated antibody, which is an antibody without Fc sugar expression, for example by mutation of one or more residues that encode a glycosylated type or by expression in an organism such as a bacterium in which sugars are not attached to proteins.

"IgG" used herein means a polypeptide belonging to the class of antibodies, which is substantially encoded by a recognized immunoglobulin gamma gene. In humans, IgG includes IgG1, IgG2, IgG3 and IgG4. In mice, IgG includes IgG1, IgG2a, IgG2b, and IgG3. "Immunoglobulin (Ig)" herein means a protein consisting of one or more polypeptides substantially encoded by immunoglobulin genes. Immunoglobulins include, but are not limited to, antibodies. Immunoglobulins can have many structural types, including but not limited to full-length antibodies, antibody fragments, and single immunoglobulin domains. Known Ig domains in IgG antibodies include VH, Cγi, Cγ2, Cγ3, VL and CL.

An "antigen" used herein means a compound, composition or substance that can stimulate the production of antibodies or a T cell response in an animal, including compositions that are injected or absorbed into the animal, which can be proteins, carbohydrates, lipids or other pathogens.

A "single domain antibody" used herein means an immunoglobulin variable domain capable of specifically binding an antigenic epitope without pairing with other immunoglobulin variable domains.

A "variable region of a heavy chain (VH)" used herein means a region of a heavy chain of immunoglobulin that varies widely in an amino acid sequence near a N-terminus, which is a variable domain of a heavy chain in a conventional 4-chain antibody. The variable region of the heavy chain (VH) is especially used to distinguish VHH antibodies of Camelidae.

"Identity" used herein means a similarity between nucleotide or amino acid sequences, or is referred to as sequence identity. The sequence identity is often measured in terms of percent identity (or similarity or homology); the higher the percentage, the more similar two sequences are. Homologs or variants will have a relatively high degree of sequence identity when aligned by using a standard method. Sequence alignment methods for comparison are well known in the art. Various programs and alignment algorithms are described in: Smith and Waterman, Adv Appl. Math., 2:482, 1981; Needlema and Wunsch, J. Mol.Biol., 48: 443, 1970; Pearson and Lipman, Proc. Natl. Acad. Sci. U.S.A. 85: 2444, 1988; Higgins and Sharp, Gene 73: 237-244, 1988; Higgins and Sharp, CABIOS 5: 151-153, 1989; Corpet et al., Nucleic Acids Research 16: 10881-10890, 1988; and Altschul et al., Nature Genet., 1994, 6: 119-129.

A NCBI Basic Local Alignment Search Tool (BLAST TM) (Altschul et al., J. Mol. Biol., 215: 403-410, 1990.) can be obtained from several sources, including the National Center for Biotechnology Information (NCBI, Bethesda, Md.) and programs blastp, blastn, blastx, tblastn and tblastx for sequence analysis on the internet.

"Fully human" used herein means that antibodies are all encoded by human antibody genes.

A "fusion protein" used herein means an expression product of a fusion gene, or two or more proteins fused by a biological or chemical method.

A "conjugate" used herein means that an antibody or a protein molecule is linked to another small molecule or macromolecule by a biological or chemical method.

"Amino acid" used herein means one of 20 naturally occurring amino acids or any non-natural analog, which may be located at a specifically defined position. "Protein" herein means at least two covalently linked amino acids, which includes proteins, polypeptides, oligopeptides and peptides. Proteins can be composed of naturally occurring amino acids and peptide bonds, or synthetic peptidomimetic structures, i.e., "analogs". Thus, "amino acid" or "peptide residue" as used herein means both naturally occurring and synthetic amino acids. For example, homophenylalanine, citrulline and norleucine are considered amino acids for the purposes of the present invention. "Amino acid" also includes imino acid residues such as proline and hydroxyproline. A side chain may be in a (R) or (S) configuration. In preferred embodiments, the amino acid is present in the (S) or L-configuration. If non-naturally occurring side chains are used, non-amino acid substitutions may be used, e.g., to prevent or delay degradation in vivo.

"Polypeptide" used herein means a polymer in which monomers are amino acid residues linked together by amide bonds. When an amino acid is an α-amino acid, an L-optical isomer or a D-optical isomer may be used. The term "polypeptide" or "protein" as used herein is intended to encompass any amino acid sequence and include modified sequences such as glycoproteins. The term "polypeptide" is specifically intended to encompass naturally occurring proteins, as well as recombinantly or synthetically produced proteins.

"Nucleic acid" used herein means a polymer composed of nucleotide units (ribonucleotides, deoxyribonucleotides, related naturally occurring structural variants, and synthetic non-naturally occurring analogs thereof) through phosphodiester bonds. Thus, the term includes nucleotide polymers in which nucleotides and bonds between the nucleotides include non-naturally occurring synthetic analogs, such as, but not limited to, phosphorothioates, phosphoramidates, methyl phosphonate, chiral methyl phosphonate, 2'-O-methyl ribonucleotides, peptide nucleic acids (PNAs), and the like. For example, the polynucleotides can be synthesized by using an automated DNA synthesizer. The term "oligonucleotide" generally refers to short polynucleotides, typically no greater than about 50 nucleotides. It should be understood that when a nucleotide sequence is represented by a DNA sequence (i.e., A, T, G, and C), this also includes an RNA sequence (i.e., A, U, G, and C) in which "U" is substituted for "T".

Conventional symbols are used herein to describe nucleotide sequences: a left-hand end of a single-stranded nucleotide sequence is a 5' end; and a left-hand direction of a double-stranded nucleotide sequence is referred to as a 5' direction. A direction in which 5' to 3' nucleotides are added to a nascent RNA transcript is referred to as a direction of transcription. A DNA strand with the same sequence as mRNA is called a coding strand.

A "vector" used herein means a vector artificially constructed to accommodate laboratory operations on the basis of a natural plasmid. A nucleic acid molecule can be introduced into a host cell, thereby producing a transformed host cell. The vector may include a nucleic acid sequence that permits it to replicate in a host cell, such as an origin of replication, and may also include one or more selectable marker genes and other genetic elements known in the art.

A "host cell" used herein, also referred to as a recipient cell, refers to a host cell that receives a foreign gene in transformation and transduction (infection).

A "pharmaceutically acceptable carrier" used herein means a conventional pharmaceutically acceptable carrier. Remington's Pharmaceutical Sciences, EWMartin, Mack Publishing Co., Easton, Pa., 15th Edition (1975), describes compositions and formulations suitable for drug delivery of one or more therapeutic compounds or molecules (such as one or more antibodies), as well as additional medicaments.

An "effective prophylactic or therapeutic dose" herein means a certain amount of a particular agent sufficient to achieve the desired effect in a subject treated with the agent. A precise dose will depend on the purpose of treatment, and can be determined by those skilled in the art using known techniques. The dose may range from 0.01-100 mg/kg body weight or more, for example, 0.1, 1, 10 or 50 mg/kg body weight, preferably 1-10 mg/kg. As is well known in the art, adjustments may be necessary for antibody or Fc fusion body degradation, systemic or local drug delivery, and a new protease synthesis rate, as well as the age, body weight, general health, sex, diet, time of administration, drug interaction and severity of a condition, which can be determined by those skilled in the art through conventional experimental methods. Ideally, a therapeutically effective amount of an antibody is an amount sufficient to prevent, treat, or ameliorate an infection or disease. A therapeutically effective amount of an agent for preventing, ameliorating, and/or treating a subject will depend on the subject being treated, the type and severity of pains, and a manner of administration of a therapeutic composition.

"Encoding" used herein means the inherent characteristics of a specific nucleotide sequence in a polynucleotide, for example, a gene, cDNA, or mRNA is used as a template for synthesis of other polymers and macromolecules in a biological process having a defined nucleotide sequence, or a defined amino acid sequence and the biological properties resulting therefrom. Thus, if transcription and translation of mRNA produced by the gene produce a protein in cells or other biological systems, the gene encodes the protein. A coding strand (a nucleotide sequence of which is identical to a sequence of mRNA and is usually provided in a sequence listing) can be referred as encoding a protein and a non-coding strand (used as a template for transcription, a gene, or cDNA) can be referred to as other products of the gene or cDNA. Unless otherwise specified, "nucleotide sequences encoding amino acid sequences" include all nucleotide sequences that are degenerate from each other and encode the same amino acid sequence. Nucleotide sequences encoding proteins and RNA may include introns.

Unless otherwise specified, all technical and scientific terms used herein have the same meaning as commonly understood by those of ordinary skill in the art to which the present invention belongs. The singular terms "a," "an," and "the" include plural referents unless the context clearly dictates otherwise. It should also be understood that all base sizes or amino acid sizes, and all molecular weights or molecular weight values, given for nucleic acids or polypeptides are approximate, and are provided for description. Although methods and materials similar or equivalent to those described herein can be used in the practice or testing of the present invention, suitable methods and materials are described below. The term "comprise" means "include". All publications, patent applications, patents, and other references mentioned herein are incorporated by reference in their entirety. In case of conflict, this specification (including explanations of terms) shall prevail. In addition, the materials, methods, and examples are illustrative only and not intended to be limiting.

### Examples

Standard recombinant DNA techniques and molecular cloning techniques used in the examples are well known in the art (Ausubel, F.M., et al., Current Protocols in Molecular Biology, published by Greene Publishing Assoc. and Wiley-Interscience), and materials and methods suitable for the growth of microorganisms are well known in the art. Main chemical and biological reagents were purchased from KAPA Biosystems, New England Biolabs, TransGen Biotech, Thermo Fisher Scientific, OMEGA bio-tek, etc.

The present invention will be described in detail below with reference to specific examples.

### Example 1 Affinity maturation method of single domain antibodies against SARS-CoV-2.

Single domain antibodies sdAb1 and sdAb2 contained in a bispecific antibody used sequences in variable regions of heavy chains of single domain antibodies n3113 and n3130 (Patent application No. 202010239592.8), the binding ability to SARS-CoV-2 was then improved based on the single domain antibodies described above by using an affinity maturation method, and a specific method was as follows: amino acids in 3 complementary determinant regions (CDRs) of VH of the single domain antibodies were first subjected to site-directed mutagenesis by PCR, wherein each amino acid was subjected to 20 amino acid saturation mutations. Afterwards, the mutated fragments were constructed into a phage display vector pComb3x (Addgene, product No.: 63891) by using a method of homologous recombination. Homologous recombination products were electrotransformed into Escherichia coli electrocompetent TG1 (purchased from Lucigen), single clones were picked from ampicillin plates where cells grew overnight for ELISA characterization of the single clones, and positive clones were sent for sequencing and analyzed to obtain sequences. A positive clone plasmid was transformed into HB2151 competent cells, and single colonies were picked from ampicillin plates where the cells grew overnight, and inoculated into an SB bacterial culture medium, and expression was performed at 30°C under isopropylthiogalactoside (IPTG) induction conditions for 12-14 hours. Bacteria were harvested and single domain antibodies were purified therefrom with Ni-NTA (purchased from GE Healthcare). The neutralizing ability of these antibodies to SARS-CoV-2 was determined by a pseudovirus neutralization system. The results showed that after affinity maturation of n3113, the neutralizing ability of n3113.4 was greatly improved, with an IC50 value from 10.96 µg/ml to 1.82 µg/ml. After affinity maturation of n3130, n3130.6 had the best neutralizing activity, with an IC50 value of 0.7 µg/ml, and its parental single domain antibody n3130 had a neutralizing IC50 of 4 µg/ml (data from the patent No. 202010239592.8).

### Example 2 Synthesis of bispecific antibody genes

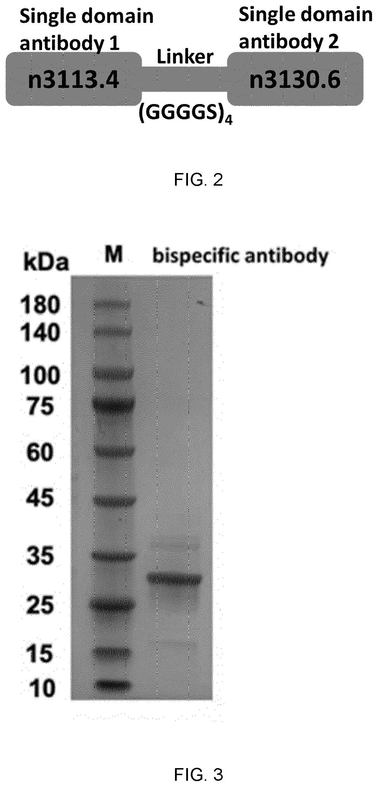

A nucleic acid sequence encoding a single domain antibody n3113.4 after affinity maturation against SARS-CoV-2 as shown in SEQ ID NO: 4 and a nucleic acid sequence encoding a single domain antibody n3130.6 after affinity maturation against SARS-CoV-2 as shown in SEQ ID NO: 5 were fused with a peptide linker (GGGGS)4 (shown in SEQ ID NO: 6), i.e., n3113.4-(GGGGS)-n3130.6, and this gene was synthesized by Genscript. A structural schematic diagram is shown in FIG. 2.

### Example 3 Construction of a bispecific antibody expression vector

The synthesized bispecific antibody genes were cloned into a prokaryotic expression vector pComb3x (Addgene, product No.: 63891) by homologous recombination. The homologous recombination products were transformed into competent Escherichia coli DH5a, and single colonies were picked for sequencing to obtain a bispecific antibody expression vector with a correct sequence.

### Example 4 Expression and purification of a bispecific antibody

The bispecific antibody expression vector was transformed into Escherichia coli HB2151 competent cells, and single colonies were picked from ampicillin plates where the cells grew overnight, and inoculated into an SB bacterial culture medium, and expression was performed at 30°C under isopropylthiogalactoside (IPTG) induction conditions for 12-14 hours. Bacteria were harvested and the bispecific antibody was purified therefrom with Ni-NTA (purchased from GE Healthcare).

### Example 5 Detection of the purity of a bispecific antibody by polyacrylamide gel electrophoresis (SDS-PAGE)

10 µg of the purified bispecific antibody was added to a loading buffer at a volume ratio of 1:6, boiling was performed in boiling water for 10 min, cooling was performed, and then transient centrifugation was performed. Electrophoresis was perfromed at a constant voltage of 120 V for 1.5 h. After the electrophoresis, a gel was gently taken down, staining was performed in an appropriate amount of 0.25% Coomassie Brilliant blue staining solution for 30 min, and destaining was performed in double distilled water until clear bands appeared. As shown in FIG. 3, the molecular weight of the bispecific antibody is about 30 kD, the bands are single, and the purity is good.

### Example 6 Binding ability of a bispecific antibody to an RBD protein of a SARS-CoV-2 variant

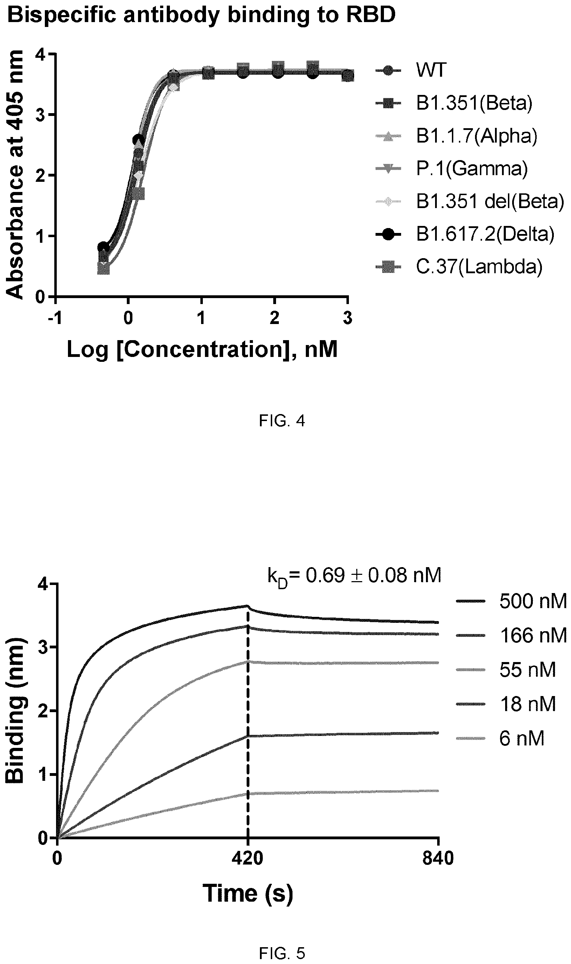

An ELISA plate was coated with 200ng of an RBD protein of a SARS-CoV-2 variant (both purchased from SinoBiological), the ELISA plate was placed overnight at 4°C, a bispecific antibody diluted in a concentration gradient was added, incubation was performed, and the binding ability of the antibody to the RBD protein of SARS-CoV-2 was tested with an anti-FLAG-HRP antibody (purchased from SIGMA). The results showed that the specific antibody could bind very strongly to the RBD protein of SARS-CoV-2 (FIG. 4), and the binding ability to each variant was equivalent to that of an original strain, indicating that the bispecific antibody had a very broad spectrum.

### Example 7 Detection of binding kinetics of a bispecific antibody to an RBD protein of a SARS-CoV-2 variant B1.617

The binding activity of a bispecific antibody to an RBD protein of a SARS-CoV-2 variant B1.617 was determined by using a BLI technology (Cell Host Microbe, 2020; 27 (6): 891-898.e5). A B1.617-RBD-his protein (purchased from SinoBiological, product No.: 40592-V08H88) was immobilized on an amino-coupled sensor (an AR2G biosensor) (Pall Fortebio) and a concentration gradient-diluted antibody solution was used as an analytical sample. The steps were as follows: the RBD protein was diluted to 10 µg/ml with a sodium acetate solution (pH 5.0), then immobilized onto the AR2G biosensor, and bound to a 3-fold gradient-diluted antibody solution (a concentration range 500-6 nM) in a running buffer (PBST) with no antibody added to blank control wells. Program settings were as follows: Association, 420s; Dissociation, 420s, and a temperature set to be 37°C. Data was analyzed by using ForteBio Data analysis 10.1 software. The background was subtracted by using the corresponding blank control wells as a control, and a curve for each concentration was fitted in a 1:1 binding mode, resulting in kinetic analysis results and curves (FIG. 5), and the results showed that the affinity of a double antibody to the RBD protein of the variant B1.617 was 0.69 nM.

### Example 8 Detection of the neutralizing activity of a bispecific antibody by a pseudovirus system

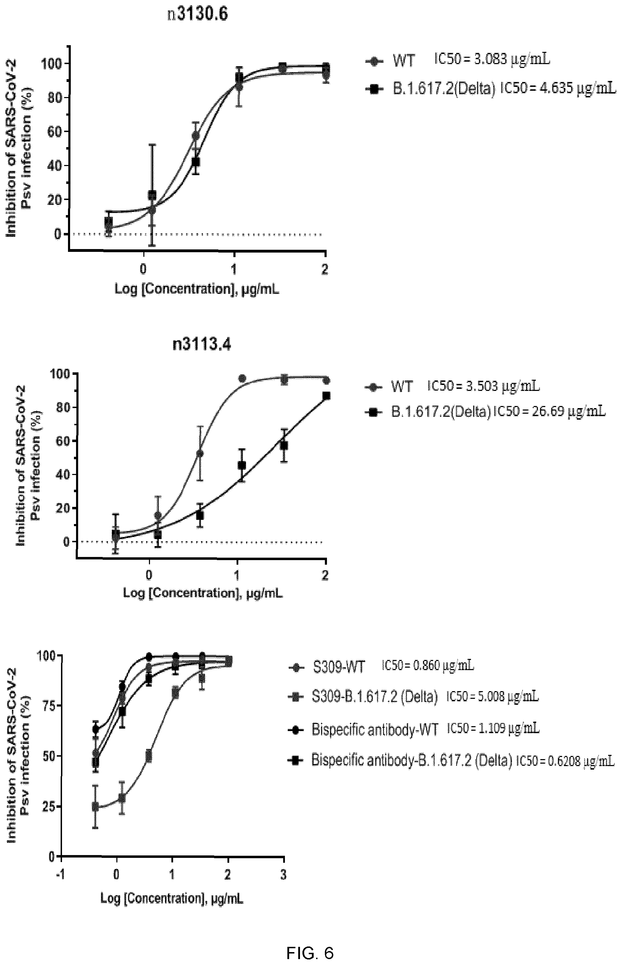

293T cells were co-transfected with an HIV-1-deleted backbone plasmid carrying a luciferase reporter gene and a recombinant plasmid of spike glycoprotein of SARS-CoV-2 or spike glycoprotein of a variant, and a supernatant was harvested after 48 h. n3113.4, n3130.6 as well as a bispecific antibody were diluted with a cell culture medium containing 10% serum, wherein a monoclonal antibody S309 with a broad-spectrum neutralizing activity against coronavirus (Nature. 2020; 583 (7815): 290-295.) was used as a positive control, and 50 µl of the diluted antibody was then mixed with 50 µl of virus to be added into Huh-7 cells. After incubation at 37°C for 12 h, the medium was replaced with a fresh medium, and incubation was continued to be performed for 48 h. A relative fluorescence value in a cell lysate was measured by using a Luciferase Assay Kit of Promega. The results showed that the neutralizing activity of the bispecific antibody against the original strain of SARS-CoV-2 and the variant strain was higher than that of the single domain antibody alone, and the neutralizing activity of the bispecific antibody against the variant strain was higher than that of the broad-spectrum antibody S309 (FIG. 6).

Specific contents of a sequence listing of the present invention are as follows:

### Sequence listing

>SEQ ID NO: 1 (n3113.4 Amino acid sequence of a variable region of a heavy chain) 
EVQLVESGGGLVQPGGSLRLSCAASDSSFYDYEMSWVRQVPGKTPEWIGSMYPS
GRTYINPSLKSLVTISRDNSENMLYLQMNSLRAEDTAMYYCVSNWASGSTGDYWG
QGTLVTVSS
SEQ ID NO: 2 (n3130.6 Amino acid sequence of a variable region of a heavy chain) 
EVQLVESGGGLVQPGGSLRLSCAASDFYFDYYEMSWVRQAPGQGLEWVSTISGLG
GATYYADSVKGRFTISRDNSKNTLYLQMNSLRAEDTALYYCATRSPFGDYAFSYWG
QGTLVTVSS
SEQ ID NO: 3 (Amino acid sequence of a peptide linker)
   GGGGSGGGGSGGGGSGGGGS
>SEQ ID NO: 4 (n3113.4 Nucleic acid sequence of a variable region of a heavy chain) 
gaagttcagctggttgaatctggtggtggtctggtacagccaggtggtagcctgcgtctgagctgtgcagcgagcgata
gcagcttctatgactatgaaatgtcctgggtacgtcaagttccaggtaagaccccggaatggatcggcagcatgtacc
cgagcggccgtacttacatcaacccgtctctgaaatccctggttaccatcagccgtgataactctgaaaacatgctgtat
ctgcagatgaactccctgcgcgctgaagataccgcaatgtattactgcgtgagcaactgggcatccggctctaccggt
gactactggggtcagggcaccctggtcactgtatcttct
>SEQ ID NO: 5 (n3130.6 Nucleic acid sequence of a variable region of a heavy chain) 
gaagtgcagctggtagaatctggcggcggtctggttcaaccaggcggttctctgcgcctgtcttgtgctgcttctgacttct
actttgattactacgaaatgagctgggttcgtcaggctccaggtcagggtctggaatgggtatctactatctccggtctgg
gcggcgctacctattacgcggatagcgttaaaggccgtttcacgatcagccgtgacaactctaaaaacactctgtacct
gcagatgaacagcctgcgtgcagaagacactgcactgtattactgtgctacccgtagcccgtttggtgattacgcgttct
cctactggggccagggcactctggttaccgttagcagc
>SEQ ID NO: 6 (Nucleic acid sequence of a peptide linker)
   ggtggcggtggttctggtggtggcggttctggtggtggtggttctggtggtggtggtagc

The above description of the disclosed examples enables those skilled in the art to implement or use the present invention. The above are only the preferred embodiments of the present invention, and it should be noted that for those skilled in the art, several modifications and supplements can be made without departing from the principles of the present invention, and these modifications and supplements should also be regarded as the scope of protection of the present invention. Various modifications to these examples will be obvious to those skilled in the art, and the generic principles defined herein may be implemented in other examples without departing from the spirit or scope of the present invention. Thus, the present invention will not be limited to the examples shown herein but is intended to conform to the widest scope consistent with the principles and novel features disclosed herein.

## Claims

1. A bispecific antibody that binds to SARS-CoV-2, **characterized by** comprising two single domain antibodies sdAb-1 and sdAb-2 that can bind to spike glycoprotein of SARS-CoV-2, wherein the sdAb-1 has an amino acid sequence shown in SEQ ID NO: 1, and the sdAb-2 has an amino acid sequence shown in SEQ ID NO: 2.

2. The bispecific antibody according to claim 1, **characterized by** comprising the amino acid sequence shown in SEQ ID NO: 1 and/or SEQ ID NO: 2 or a sequence having at least 90%, 91%, 92%, 93%, 94%, 95%, 96%, 97%, 98% or 99% identity to the amino acid sequence.

3. The bispecific antibody according to claim 1, **characterized in that** a C-terminus of the sdAb-1 is linked to a N-terminus of the sdAb-2 by a peptide linker.

4. The bispecific antibody according to claim 3, **characterized in that** the peptide linker has a sequence of (GGGGS)4, as shown in SEQ ID NO: 3.

5. The bispecific antibody according to any one of claims 1-4, **characterized in that** each single domain antibody is a variable region of a heavy chain (VH).

6. The bispecific antibody according to claim 5, **characterized in that** the variable region of the heavy chain (VH) is fully human.

7. A fusion protein, **characterized by** comprising the bispecific antibody according to any one of claims 1-6 fused to a heterologous protein.

8. The fusion protein according to claim 7, **characterized in that** the heterologous protein is Fc of human immunoglobulin.

9. The fusion protein according to claim 8, **characterized in that** the heterologous protein is Fc of human IgG1.

10. A conjugate, **characterized by** being formed by conjugating the bispecific antibody according to any one of claims 1-6, or the fusion protein according to any one of claims 7-9 with an effector molecule.

11. The conjugate according to claim 10, **characterized in that** the effector molecule is a detectable label.

12. The conjugate according to claim 11, **characterized in that** the detectable label is a fluorescent label, a radiolabel, avidin, biotin, or an enzyme.

13. A nucleic acid molecule, **characterized by** encoding the bispecific antibody according to any one of claims 1-6, or the fusion protein according to any one of claims 7-9.

14. A vector, **characterized by** comprising the nucleic acid molecule according to claim 13.

15. A host cell, **characterized by** comprising the nucleic acid molecule according to claim 13 or the vector according to claim 14.

16. A pharmaceutical composition, **characterized by** comprising an effective prophylactic or therapeutic dose of the bispecific antibody according to any one of claims 1-6, or the fusion protein according to any one of claims 7-9, or the conjugate according to any one of claims 10-11, or the nucleic acid molecule according to claim 13, or the vector according to claim 14, and a pharmaceutically acceptable carrier.

17. Use of the bispecific antibody according to any one of claims 1-6, or the fusion protein according to any one of claims 7-9, or the conjugate according to any one of claims 10-11, or the nucleic acid molecule according to claim 13, or the vector according to claim 14, or the pharmaceutical composition according to claim 16 in the preparation of drugs for treating or preventing diseases caused by SARS-CoV-2 infection.

18. Use of the bispecific antibody according to any one of claims 1-6, or the fusion protein according to any one of claims 7-9, or the conjugate according to any one of claims 10-11, or the nucleic acid molecule according to claim 13, or the vector according to claim 14, or the pharmaceutical composition according to claim 16 in the preparation of a diagnostic product for diagnosing SARS-CoV-2 infection or a detection product for detecting SARS-CoV-2 infection.
